# EUROPÄISCHE PATENTSCHRIFT

(11) **EP 1 611 206 B1**
(45) Veröffentlichungstag und Bekanntmachung des Hinweises auf die Patenterteilung: **02.04.2008**
(21) Anmeldenummer: 04713856.5
(22) Anmeldetag: 24.02.2004
(51) Int. Cl.: C09B 57/00

(54) **HETEROCYCLISCHE FARBMITTEL AUF BASIS VON BENZODIPYRROLEN**
HETEROCYCLIC DYES BASED ON BENZODIPYRROLES
COLORANT HETEROCYCLIQUE A BASE DE BENZODIPYRROLES

(30) Priorität: 27.03.2003 DE 10313701
(43) Veröffentlichungstag der Anmeldung: 04.01.2006
(73) Patentinhaber: Clariant Produkte (Deutschland) GmbH, 65929 Frankfurt am Main (DE)
(72) Erfinder: HECKMANN, Heino, 65835 Liederbach (DE); WEISS, Andre, 86159 Augsburg (DE)
(86) Internationale Anmeldenummer: PCT/EP2004/001799
(87) Internationale Veröffentlichungsnummer: WO 2004/085542

(56) Entgegenhaltungen:
- DE-A- 2 236 629
- DE-A- 3 911 643
- US-A- 4 122 087
- CLOSS ET AL: "2,6-Diaza-s-indcenes" ANGEWANDTE CHEMIE INTERNATIONAL EDITION ENGLAND, Bd. 10, 1987, Seiten 1037-1039, XP002282814 DE in der Anmeldung erwähnt

## Beschreibung

Die vorliegende Erfindung betrifft neue organische Pigmente und Farbstoffe.

Auf dem Gebiet der Farbmittel besteht eine ständige Marktnachfrage nach neuen Farbnuancen, die hohe Migrations- und Lichtechtheiten, gute Wärmestabilitäten und eine hohe Färbekraft, sowie im Falle von Pigmenten zusätzlich hohe Lösungsmittelechtheiten aufweisen.

In Angew. Chem. 1987, 99(10), Seiten 1068-1070, werden salzartige Verbindungen beschrieben, die das Dianion des 2,2',2",2"'-Benzo[1,2-c:4,5-c']dipyrrol-1,3,5,7(2H,6H)-tetraylidentetrakis-propandinitrils enthalten. Diese Verbindungen und ihre Edukte werden in einer aufwändigen Synthesefolge erhalten. Für eine Anwendung als Farbmittel eignen sich diese Verbindungen aufgrund ihrer geringen Echtheiten nicht.

Es bestand die Aufgabe, neue Farbmittel zum Einfärben oder Pigmentieren von organischen oder anorganischen, hoch- oder niedermolekularen, insbesondere hochmolekularen organischen Materialien zu finden, die von einfach zugänglichen Zwischenprodukten ausgehen.

Es wurde gefunden, dass diese Aufgabe überraschenderweise durch Verbindungen der Formel (I) gelöst wird.

Ein Gegenstand der vorliegenden Erfindung sind somit Verbindungen der allgemeinen Formel (I) 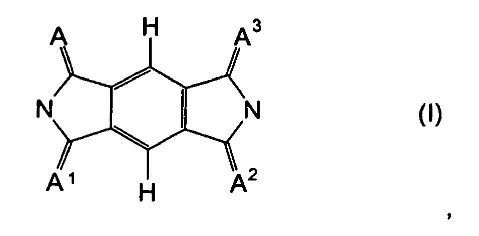 worin A für den zweiwertigen Rest einer cyclischen Verbindung der allgemeinen Formel (II) 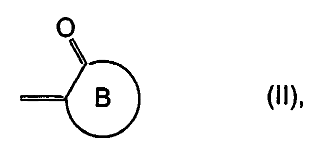 wobei B eine alicyclische oder heterocyclische Gruppe bedeutet, und A¹, A² und A³ gleich oder verschieden sind und die Bedeutung von A haben oder =NR bedeuten, wobei R Wasserstoff, unsubstituiertes oder durch 1, 2, 3 oder 4 Reste Halogen, R⁰, OR⁰, SR⁰, NH₂, NHR⁰, NR⁰₂, NO₂, COOH, COOR⁰, CONH₂, CONHR⁰, CONR⁰₂, CN, SO₃H, SO₂(OR⁰), SO₂R⁰, oder durch einen 5- bis 7-gliedrigen heteroaromatischen Rest mit 1, 2 oder 3 Heteroatomen aus der Gruppe N, O und S substituiertes Phenyl; einen 5- bis 7-gliedrigen heteroaromatischen Rest mit 1, 2 oder 3 Heteroatomen aus der Gruppe N, O und S; NH₂, NHR⁰, NR⁰₂, NHCONH₂ oder NHCONHR⁰ bedeutet, wobei R⁰ für C₁-C₁₈-Alkyl oder C₆-C₂₄-Aryl steht.

Bevorzugt sind Verbindungen der Formel (I), worin einer der Reste A¹, A² und A³, besonders bevorzugt A² oder A³, die Bedeutung von A hat.
Bevorzugt sind weiterhin Verbindungen der Formel (I), worin zwei der Reste A¹, A² und A³, besonders bevorzugt A¹ und A², die Bedeutung von A haben.
Bevorzugt sind weiterhin Verbindungen der Formel (I), worin A¹, A² und A³ die Bedeutung von A haben.

Besonders bevorzugt sind Verbindungen der allgemeinen Formel (I), worin A für den zweiwertigen Rest einer cyclischen Verbindung der allgemeinen Formel (III) steht 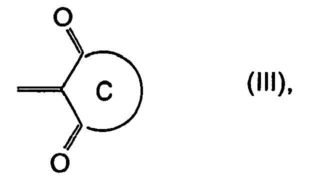 wobei C eine alicyclische oder heterocyclische Gruppe bedeutet.

Ganz besonders bevorzugt sind Verbindungen der allgemeinen Formel (I), worin A einen zweiwertigen Rest der Formeln (a) bis (g) bedeutet, 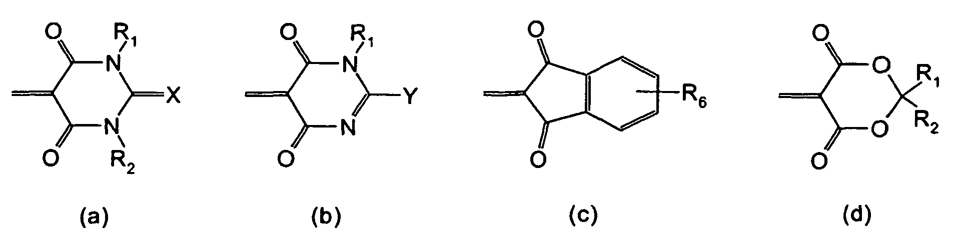 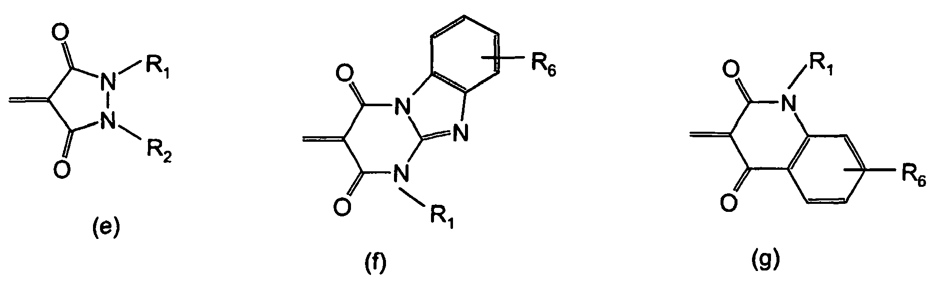 wobei R₁ und R₂ unabhängig voneinander für Wasserstoff, C₁-C₂₅-Alkyl, C₅-C₁₂-Cycloalkyl, C₆-C₂₄-Aryl, C₁-C₂₅-Alkyl-(C₆-C₁₀-aryl), einen 5- bis 7-gliedrigen heteroaromatischen Rest mit 1, 2 oder 3 Heteroatomen aus der Gruppe N, O und S, -(CH₂)ₙ-COR₃ oder -(CH₂)ₘ-OR₄, stehen,
worin R₃ für Hydroxy, Amino oder für unsubstituiertes oder ein- oder mehrfach, z.B. 1-, 2-, 3- oder 4-fach, mit Hydroxy oder Amino substituiertes C₁-C₂₅-Alkoxy, C₁-C₂₅-Alkylamino, Di-(C₁-C₂₅-alkyl)-amino, C₆-C₂₄-Arylamino, Di-(C₆-C₂₄-Aryl)-amino, C₁-C₂₅-Alkyl-(C₆-C₁₀-aryl)-amino oder C₂-C₂₄-Alkenyloxy steht;
R₄ für Wasserstoff oder -CO-(C₁-C₂₅-Alkyl) steht, und
n und m unabhängig voneinander für eine ganze Zahl von 0 bis 6, bevorzugt 1 bis 4 stehen,
und worin in R₁, R₂, R₃ und R₄ eine C-C- Einheit auch durch eine Ethereinheit C-O-C ersetzt sein kann;
X für =O, =S oder =NR₅ steht, worin R₅ die gleiche Bedeutung wie R₁ oder R₂ hat; Y für Wasserstoff, R₂, OR₂, SR₂, NHCN oder NR₂R₅ steht;
und R₆ Wasserstoff, Halogen, CN, R₂, OR₂, SR₂, NR₂R₅, NO₂, SO₂(OR₂), SO₂R₂, SO₂NR₂R₅ oder PO₂(OR₂) bedeutet.

R₁ und R₂ sind besonders bevorzugt Wasserstoff, C₁-C₁₈-Alkyl, C₅-C₆-Cycloalkyl, C₆-C₁₀-Aryl, Benzyl, Pyridyl, Pyrryl, Thienyl, Imidazolyl, Oxazolyl, Thiazolyl, Pyrimidyl, Hydroxycarbonyl-C₀-C₆-alkyl, C₁-C₁₈-Alkoxycarbonyl-C₀-C₆-alkyl, Aminocarbonyl-C₀-C₆-alkyl, C₁-C₁₈-Alkylaminocarbonyl-C₀-C₆-alkyl, C₆-C₁₀-Arylaminocarbonyl-C₀-C₆-alkyl, Di(C₁-C₁₈-alkyl)-aminocarbonyl-C₀-C₆-alkyl, C₁-C₁₈-Alkyl-C₆-C₁₀-arylaminocarbonyl-C₀-C₆-alkyl und Di(C₆-C₁₀-aryl)-aminocarbonyl-C₀-C₆-alkyl.

R₃ ist besonders bevorzugt Hydroxy, C₁-C₁₈-Alkoxy, C₁-C₁₈-Alkylamino, Di(C₁-C₁₈-alkyl)-amino, Benzylamino, C₆-C₁₀-Arylamino, Di(C₆-C₁₀-aryl)-amino oder (C₂-C₁₈)-Alkenyloxy.

R₆ ist besonders bevorzugt Wasserstoff, Cl, Br, C₁-C₁₈-Alkyl, C₅-C₆-Cycloalkyl, Benzyl, C₆-C₁₀-Aryl, Pyridyl, Pyrryl, Thienyl, Imidazolyl, Oxazolyl, Thiazolyl, Pyrimidyl, C₁-C₁₈-Alkoxy, C₆-C₁₀-Aryloxy, C₁-C₁₈-Alkylthio, C₆-C₁₀-Arylthio, C₁-C₁₈-Alkylamino, C₆-C₁₀-Arylamino, Di(C₁-C₁₈-alkyl)-amino, C₁-C₁₈-Alkyl-C₆-C₁₀-arylamino, Di(C₆-C₁₀-aryl)-amino, SO₃H, C₁-C₁₈-Alkoxysulfonyl, C₁-C₁₈-Alkylsulfonyl und Di (C₁-C₁₈-alkyl)-aminosulfonyl.

Ein weiterer Gegenstand der vorliegenden Erfindung ist ein Verfahren zur Herstellung von Verbindungen der allgemeinen Formel (I) durch Umsetzung von 1,2,4,5-Tetracyanobenzol mit mindestens 2 Equivalenten Ammoniak und/oder Alkoholaten MOR₇, worin M Natrium oder Kalium bedeutet, R₇ für C₁-C₁₈-Alkyl oder -(CH₂)ₘ-OH steht, und m eine ganze Zahl von 1 bis 6 bedeutet, und eine C-C-Einheit auch durch eine Ethereinheit C-O-C ersetzt sein kann, wie z.B. Natriummethylat, Natriumethylat, Natriumamylat, Kaliummethylat oder Kalium-tert.-butylat, zu tetra-, tri-, di- oder monoiminosubstituierten Benzodipyrrolen, die 0 bis 5 Alkoxysubstituenten tragen können, beispielhaft repräsentiert durch Verbindungen der Formeln (IV), (V) oder (VI) 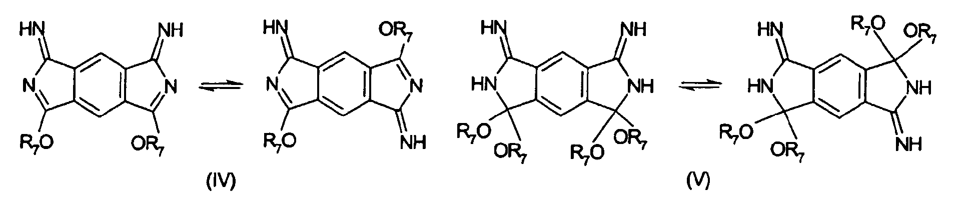 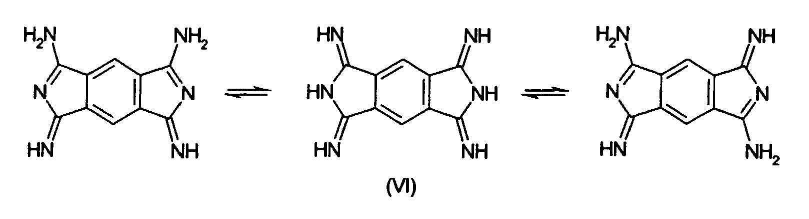 in einem Lösemittel oder Lösemittelgemisch unter basischen bis neutralen Bedingungen bei einer Temperatur von -20 bis 120°C, bevorzugt 0 bis 100°C, besonders bevorzugt 20 bis 80°C,
die anschließend nach Zwischenisolierung oder ohne zwischenisoliert zu werden in einem Lösungsmittel oder Lösungsmittelgemisch unter neutralen bis sauren Bedingungen, vorzugsweise in Gegenwart einer organischen Säure, wie z.B. Ameisensäure, Essigsäure und Propionsäure, oder einer anorganischen Säure, wie z.B. Schwefelsäure, Salzsäure und Phosphorsäure, und zweckmäßigerweise bei einer Temperatur von 10 bis 250°C, insbesondere 20 bis 200°C, besonders bevorzugt 30 bis 150°C, mit mindestens 1 Equivalent einer cyclischen Verbindung der Formel (VII), 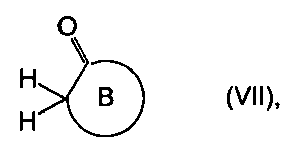 und ggf. höchstens 3 Equivalenten H₂NR umgesetzt werden, wobei R die vorstehende Bedeutung hat.

Zweckmäßigerweise erfolgt die erfindungsgemäße Umsetzung in Gegenwart eines Lösemittels wie Methanol, Ethanol, Glykolen, Dichlormethan, Chloroform, Dimethylformamid, N-Methyl-pyrrolidon, Toluol, Mono-, Di- oder Trichlorbenzol oder einer Mischung davon.

Erfindungsgemäße Verbindungen der allgemeinen Formel (I) werden zum Einfärben oder Pigmentieren von organischen oder anorganischen, hoch- oder niedermolekularen, insbesondere hochmolekularen organischen Materialien verwendet.

Je nach Art ihrer Substituenten und des zu färbenden hochmolekularen organischen Materials können die erfindungsgemäßen Verbindungen als polymerlösliche Farbstoffe oder als Pigmente verwendet werden. Im letzteren Fall ist es vorteilhaft, die bei der Synthese anfallenden Produkte (Rohpigmente) durch Nachbehandlung in organischen Lösungsmitteln und bei erhöhten Temperaturen, beispielsweise bei 60 bis 200°C, insbesondere bei 70 bis 150°C, vorzugsweise bei 75 bis 100°C, in eine feindisperse Form mit oft weiter verbesserten Pigmenteigenschaften zu überführen. Die Nachbehandlung wird vorzugsweise mit einer Mahl- oder Knetoperation kombiniert.

Die erfindungsgemäßen Farbmittel eignen sich ausgezeichnet zum Färben von hochmolekularen Materialien, die organischer oder anorganischer Natur sein können, und Kunststoffe und/oder Naturstoffe bedeuten. Es kann sich zum Beispiel um Naturharze, trocknende Öle, Kautschuk oder Casein handeln. Es kann sich aber auch um abgewandelte Naturstoffe handeln, wie beispielsweise Chlorkautschuk, ölmodifizierte Alkydharze, Viskose, Cellulosederivate, wie Celluloseester oder Celluloseether, und insbesondere um vollsynthetische organische Polymere (Kunststoffe), die durch Polymerisation, Polykondensation oder Polyaddition erhalten werden können. Aus der Klasse der durch Polymerisation hergestellten Kunststoffe seien besonders folgende genannt: Polyolefine, wie zum Beispiel Polyethylen, Polypropylen, Polyisobutylen, und substituierte Polyolefine, wie beispielsweise Polystyrol, Polyvinylchlorid, Polyvinylidenchlorid, Polyvinylacetale, Polyacrylnitril, Polyacrylsäure, Polymethacrylsäure, Polyacrylsäure- und Polymethacrylsäureester oder Polybutadien, sowie Copolymerisate davon.
Aus der Klasse der durch Polyaddition und Polykondensation hergestellten Kunststoffe seien genannt: Polyester, Polyamide, Polyimide, Polycarbonate, Polyurethane, Polyether, Polyacetale, sowie die Kondensationsprodukte von Formaldehyd mit Phenolen (Phenoplaste) und die Kondensationsprodukte von Formaldehyd mit Harnstoff, Thioharnstoff und Melamin (Aminoplaste). Weiterhin kann es sich auch um Silikone oder Silikonharze handeln.

Solche hochmolekularen Materialien können einzeln oder in Gemischen als plastische Massen, Schmelzen oder in Form von Spinnlösungen vorliegen. Sie können auch in Form ihrer Monomeren oder im polymerisierten Zustand in gelöster Form als Filmbildner oder Bindemittel für Lacke oder Druckfarben vorliegen, wie Leinölfirnis, Nitrocellulose, Alkydharze, Melaminharze und Formaldehydharze oder Acrylharze.

Die erfindungsgemäßen Verbindungen eignen sich demzufolge als Farbmittel in Anstrichfarben auf öliger oder wässriger Grundlage, in Lacken verschiedener Art, Tarnfarben, zum Spinnfärben, zum Massefärben oder Pigmentieren von Kunststoffen, in Druckfarben für das graphische Gewerbe, wie zum Beispiel im Papier-, Textil- oder Dekorationsdruck, und in der Papiermassefärbung, zur Herstellung von Tinten, Ink-Jet Tinten auf wässriger oder nichtwässriger Basis, Mikroemulsionstinten und Tinten, die nach dem Hot-melt Verfahren arbeiten.

Die erfindungsgemäßen Verbindungen sind auch geeignet als Farbmittel in elektrophotographischen Tonern und Entwicklern, wie z.B. Ein- oder Zweikomponentenpulvertonern (auch Ein- oder Zweikomponenten-Entwickler genannt), Magnettoner, Flüssigtoner, Latextoner, Polymerisationstoner sowie Spezialtoner.

Typische Tonerbindemittel sind Polymerisations-, Polyadditions- und Polykondensationsharze, wie Styrol-, Styrolacrylat-, Styrolbutadien-, Acrylat-, Polyester-, Phenol-Epoxidharze, Polysulfone, Polyurethane, einzeln oder in Kombination, sowie Polyethylen und Polypropylen, die noch weitere Inhaltsstoffe, wie Ladungssteuermittel, Wachse oder Fließhilfsmittel, enthalten können oder im Nachhinein mit diesen Zusätzen modifiziert werden.

Des weiteren sind die erfindungsgemäßen Verbindungen geeignet als Farbmittel in Pulver und Pulverlacken, insbesondere in triboelektrisch oder elektrokinetisch versprühbaren Pulverlacken, die zur Oberflächenbeschichtung von Gegenständen aus beispielsweise Metall, Holz, Kunststoff, Glas, Keramik, Beton, Textilmaterial, Papier oder Kautschuk zur Anwendung kommen.

Als Pulverlackharze werden typischerweise Epoxidharze, carboxyl- und hydroxylgruppenhaltige Polyesterharze, Polyurethan- und Acrylharze zusammen mit üblichen Härtern eingesetzt. Auch Kombinationen von Harzen finden Verwendung. So werden beispielsweise häufig Epoxidharze in Kombination mit carboxyl- und hydroxylgruppenhaltigen Polyesterharzen eingesetzt. Typische Härterkomponenten (in Abhängigkeit vom Harzsystem) sind beispielsweise Säureanhydride, Imidazole sowie Dicyandiamid und deren Abkömmlinge, verkappte Isocyanate, Bisacylurethane, Phenol- und Melaminharze, Triglycidylisocyanurate, Oxazoline und Dicarbonsäuren.

Außerdem sind die erfindungsgemäßen Verbindungen als Farbmittel in Tinten, vorzugsweise Ink-Jet Tinten, wie z.B. auf wässriger oder nichtwässriger Basis, Mikroemulsionstinten sowie in solchen Tinten, die nach dem hot-melt-Verfahren arbeiten, geeignet.
Ink-Jet-Tinten enthalten im allgemeinen insgesamt 0,5 bis 15 Gew.-%, vorzugsweise 1,5 bis 8 Gew.-%, (trocken gerechnet) einer oder mehrerer der erfindungsgemäßen Verbindungen.
Mikroemulsionstinten basieren auf organischen Lösemitteln, Wasser und ggf. einer zusätzlichen hydrotropen Substanz (Grenzflächenvermittler). Mikroemulsionstinten enthalten 0,5 bis 15 Gew.-%, vorzugsweise 1,5 bis 8 Gew.-%, einer oder mehrerer der erfindungsgemäßen Verbindungen, 5 bis 99 Gew.-% Wasser und 0,5 bis 94,5 Gew.-% organisches Lösungsmittel und/oder hydrotrope Verbindung.
"Solvent based" Ink-Jet-Tinten enthalten vorzugsweise 0,5 bis 15 Gew.-% einer oder mehrerer erfindungsgemäßer Verbindungen, 85 bis 99,5 Gew.-% organisches Lösungsmittel und/oder hydrotrope Verbindungen.

Hot-Melt-Tinten basieren meist auf Wachsen, Fettsäuren, Fettalkoholen oder Sulfonamiden, die bei Raumtemperatur fest sind und bei Erwärmen flüssig werden, wobei der bevorzugte Schmelzbereich zwischen ca. 60°C und ca. 140°C liegt. Hot-Melt Ink-Jet-Tinten bestehen z.B. im wesentlichen aus 20 bis 90 Gew.-% Wachs und 1 bis 10 Gew.-% einer oder mehrerer der erfindungsgemäßen Verbindungen. Weiterhin können 0 bis 20 Gew.-% eines zusätzlichen Polymers (als "Farbstofflöser"), 0 bis 5 Gew.-% Dispergierhilfsmittel, 0 bis 20 Gew.-% Viskositätsveränderer, 0 bis 20 Gew.-% Plastifizierer, 0 bis 10 Gew.-% Klebrigkeitszusatz, 0 bis 10 Gew.-% Transparenzstabilisator (verhindert z.B. Kristallisation der Wachse) sowie 0 bis 2 Gew.-% Antioxidans enthalten sein.

Außerdem sind die erfindungsgemäßen Verbindungen auch geeignet als Farbmittel für Farbfilter, sowohl für die additive wie für die subtraktive Farberzeugung, sowie als Farbmittel für elektronische Tinten ("electronic inks" bzw. "e-inks") oder "electronic paper"("e-paper").

Bei der Herstellung sogenannter Farbfilter, sowohl reflektierender wie durchsichtiger Farbfilter, werden Pigmente in Form einer Paste oder als pigmentierte Photoresists in geeigneten Bindemitteln (Acrylate, Acrylester, Polyimide, Polyvinylalkohole, Epoxide, Polyester, Melamine, Gelantine, Caseine) auf die jeweiligen LCD-Bauteilen (z.B. TFT-LCD= Thin Film Transistor Liquid Crystal Displays oder z.B. ((S) TN-LCD = (Super) Twisted Nematic-LCD) aufgebracht. Neben einer hohen Thermostabilität ist für eine stabile Paste bzw. einem pigmentierten Photoresist auch eine hohe Pigmentreinheit Voraussetzung. Darüber hinaus können die pigmentierten Color Filter auch durch Ink Jet-Druckverfahren oder andere geeignete Druckverfahren aufgebracht werden.

Die vorliegende Erfindung betrifft überdies die Verwendung der erfindungsgemäßen Farbmittel in optischen Schichten für die optische Datenspeicherung, bevorzugt für die optische Datenspeicherung, bei der ein Laser zum Schreiben der Daten verwendet wird. Die für diese Anwendung notwendige Löslichkeit der Farbmittel im Anwendungsmedium kann durch die Art und Anzahl der Substituenten eingestellt werden.

Des weiteren eignen sich die erfindungsgemäßen Verbindungen als Farbmittel in Kosmetik, zur Einfärbung von Saatgut und zur Einfärbung von Mineralölen, Schmierfetten und Wachsen.

Je nach Art der Substituenten der erfindungsgemäßen Verbindungen, zeichnen sich die erhaltenen Färbungen durch gute Hitze-, Licht- und Wetterechtheit, Chemikalienbeständigkeit und die sehr guten applikatorischen Eigenschaften, z.B. Kristallisierechtheit und Dispergierechtheit und insbesondere durch ihre Migrier-, Ausblüh-, Überlackier- und Lösungsmittelechtheit aus. Die als polymerlösliche Farbstoffe eingesetzten Verbindungen weisen naturgemäß nur eine geringe oder eingeschränkte Lösungsmittelechtheit auf.

Einen weiteren Erfindungsgegenstand bildet eine Zusammensetzung, enthaltend ein organisches oder anorganisches, hochmolekulares oder niedermolekulares, insbesondere hochmolekulares organisches Material, und mindestens eine erfindungsgemäße Verbindung in einer färberisch wirksamen Menge, in der Regel im Bereich von 0,005 bis 70 Gew.-%, insbesondere von 0,01 bis 10 Gew.-%, bezogen auf das organische oder anorganische Material.

### Beispiele

Herstellung von Benzo[1,2-c:4,5-c']dipyrrol-1,3,5,7(2H,6H)-tetraimin

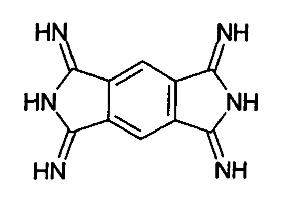

Ammoniakgas wird bei 55 - 60°C in eine Mischung von 20,0 g 1,2,4,5-Tetracyanobenzol in 500 mL Ethylenglycol eingeleitet, bis das Edukt vollständig umgesetzt ist (Kontrolle durch Dünnschichtchromatographie). Die Mischung wird auf 20°C abgekühlt und weitere 4h gerührt. Das Produkt wird abgesaugt, mit Ethylenglycol und Aceton gewaschen. Nach Trocknen bei 20°C im Vakuum werden 23,4 g (98 % d.Th.) eines beigen Pulvers der vorstehenden Formel erhalten.
MS (m/e): 213 [M+H]⁺

### Beispiel 1: 5,5',5",5"'-Benzo[1,2-c:4,5-c']dipyrrol-1,3,5,7(2H,6H)-tetraylidentetrakis-1,3-dimethyl-2,4,6-trioxo-tetrahydropyrimidin

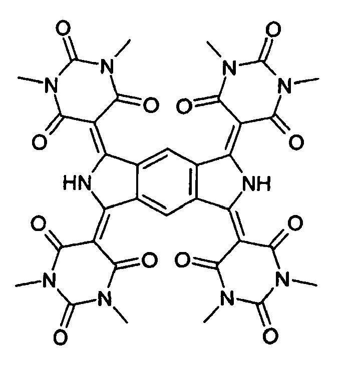

a) 5,3 g Benzo[1,2-c:4,5-c']dipyrrol-1,3,5,7(2H,6H)-tetraimin und 23,4 g 1,3-Dimethylbarbitursäure werden in 225 ml Eisessig erst 30 min bei Raumtemperatur gerührt und danach 6 Stunden zum Sieden erhitzt. Die Suspension wird auf Raumtemperatur abgekühlt, filtriert, nacheinander mit Eisessig, Methanol und heißem Wasser (ca. 90°C) gewaschen und anschließend bei 60°C getrocknet. Die Ausbeute beträgt 13,2 g (69 % d.Th.) eines orangefarbenen Pulvers der vorstehenden Formel.
   Schmelzpunkt: > 300°C
   MALDI (m/e): 768 [M-H]⁻
   H-NMR (D₂SO₄): 9.34 (s, 2H), 2.73 (s, 24H)
b) Eine Suspension von 11,3 g 1,2,4,5-Tetracyanobenzol und 3,0 g Natriummethylatlösung (30 % in Methanol) in 340 ml Methanol wird 16 Stunden bei Raumtemperatur gerührt. Nach Zugabe von 6,8 g Eisessig und 43,4 g 1,3-Dimethylbarbitursäure wird die Reaktionsmischung 24 Stunden bei Raumtemperatur und anschließend 6 Stunden zum Sieden erhitzt.
   Die Suspension wird auf Raumtemperatur abgekühlt und filtriert. Das Rohprodukt wird zur Reinigung 15 min in 250 mL Eisessig bei Siedetemperatur gerührt, heiß abgesaugt, nacheinander mit Eisessig und heißem Wasser (ca. 90°C) gewaschen und anschließend bei 60°C getrocknet. Die Ausbeute beträgt 43,2 g (89 % d.Th.) eines orangefarbenen Pulvers der vorstehenden Formel.
   Schmelzpunkt: > 300°C
   MALDI (m/e): 768 [M-H]⁻

### Beispiel 2: Umsetzung von Benzo[1,2-c:4,5-c']dipyrrol-1,3,5,7(2H,6H)-tetraimin mit Anilin und 1,3-Dimethylbarbitursäure

5,3 g Benzo[1,2-c:4,5-c']dipyrrol-1,3,5,7(2H,6H)-tetraimin und 7,0 g Anilin werden in 150 ml Eisessig 24 Stunden bei Raumtemperatur gerührt, nach Zugabe von 7,8 g 1,3-Dimethylbarbitursäure wird die Mischung 6 Stunden zum Sieden erhitzt. Die Suspension wird auf Raumtemperatur abgekühlt, filtriert, nacheinander mit Eisessig, Methanol und heißem Wasser (ca. 90°C) gewaschen und anschließend bei 60°C getrocknet. Die Ausbeute beträgt 9,26 g eines orangefarbenen Pulvers eines Gemischs der folgenden Formeln 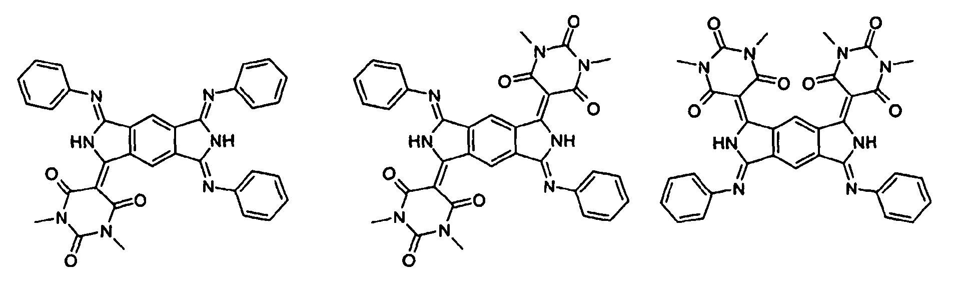 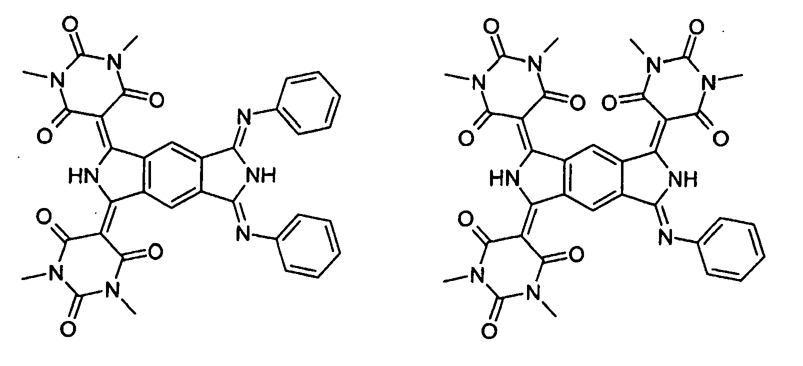 Schmelzpunkt: > 300°C
MALDI (m/e): 644, 707, 770 [M+H]⁺

### Beispiel 3: 5,5',5",5"'-Benzo[1,2-c:4,5-c']dipyrrol-1,3,5,7(2H,6H)-tetraylidentetrakis-1,3-bis-(2-ethylhexyl)-2,4,6-trioxo-tetrahydropyrimidin

1,06 g Benzo[1,2-c:4,5-c`]dipyrrol-1,3,5,7(2H,6H)-tetraimin und 8,48 g 1,3-Bis-(2-ethylhexyl)-barbitursäure werden in einer Mischung aus 90 mL Methanol und 30 mL Eisessig 24 h bei Raumtemperatur gerührt. Anschließend wird die Suspension filtriert, mit Methanol, dann heißem Wasser (ca. 90°C) gewaschen und bei 60°C getrocknet. Reinigung erfolgt durch Säulenchromatographie über Kieselgel mit einer Laufmittelmischung aus Toluol und Hexan. Die Ausbeute beträgt 1,20 g (15% d.Th.) eines roten Pulvers der folgenden Formel 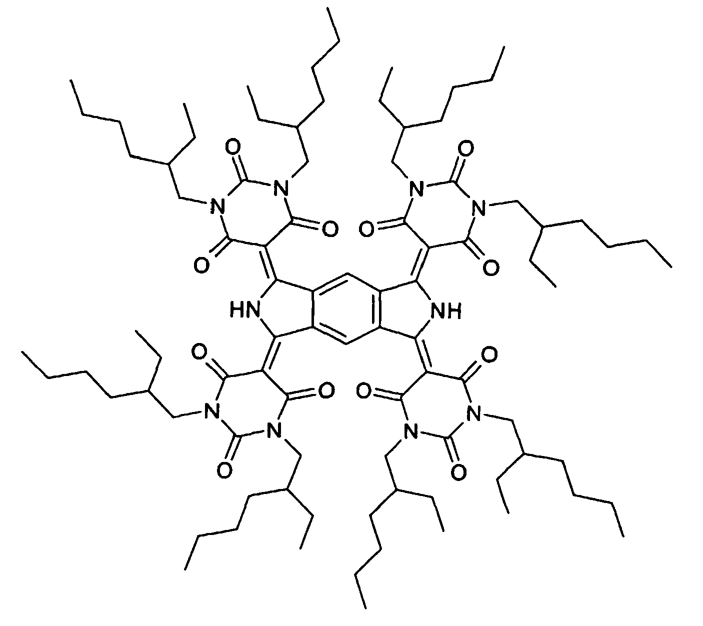 Schmelzpunkt: 113 - 115°C
MS (m/e): 1554 [M-H]⁻

### Beispiel 4: 5,5',5",5'"-Benzo[1,2-c:4,5-c']dipyrrol-1,3,5,7(2H,6H)-tetraylidentetrakis-1,3-dibutyl-2,4,6-trioxo-tetrahydropyrimidin

1,06 g Benzo[1,2-c:4,5-c']dipyrrol-1,3,5,7(2H,6H)-tetraimin und 6,01 g 1,3-Dibutylbarbitursäure werden in einer Mischung aus 10 mL Eisessig und 40 mL Toluol 24 h bei Raumtemperatur, dann 6h am Rückfluss gerührt. Nach Abkühlen wird die Suspension filtriert, mit Eisessig, Methanol und heißem Wasser (ca. 90°C) gewaschen und bei 60°C getrocknet. Die Ausbeute beträgt 4,42 g (80% d.Th.) eines roten Pulvers der folgenden Formel: 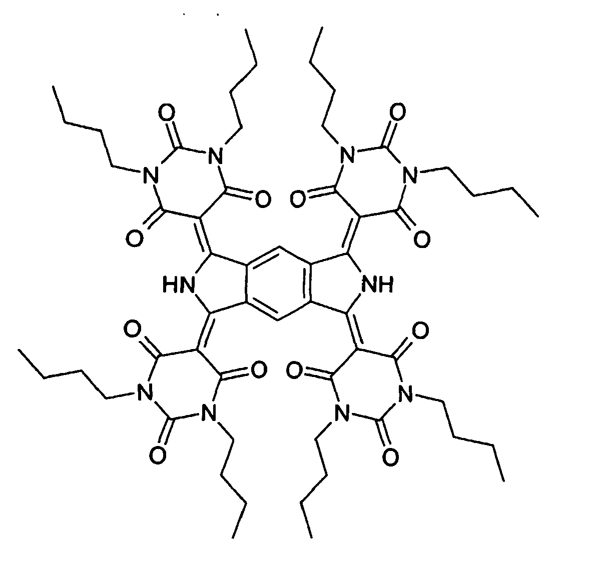 Schmelzpunkt: > 300°C
MS (m/e): 1106 [M+H]⁺

### Beispiel 5: 5,5',5",5"'-Benzo[1,2-c:4,5-c']dipyrrol-1,3,5,7(2H,6H)-tetraylidentetrakis-1,3-didodecyl-2,4,6-trioxo-tetrahydropyrimidin

1,06 g Benzo[1,2-c:4,5-c']dipyrrol-1,3,5,7(2H,6H)-tetraimin und 11,6 g 1,3-Didodecylbarbitursäure werden in einer Mischung aus 20 mL Eisessig und 80 mL Toluol 24 h bei Raumtemperatur, dann 6 h am Rückfluss gerührt. Nach Abkühlen wird die Suspension filtriert, mit Eisessig, Methanol und heißem Wasser (ca. 90°C) gewaschen und bei 60°C getrocknet. Umkristallisation kann aus Chloroform/Methanol-Gemischen erfolgen. Die Ausbeute beträgt 3,41 g (34 % d.Th.) eines roten Pulvers der folgenden Formel 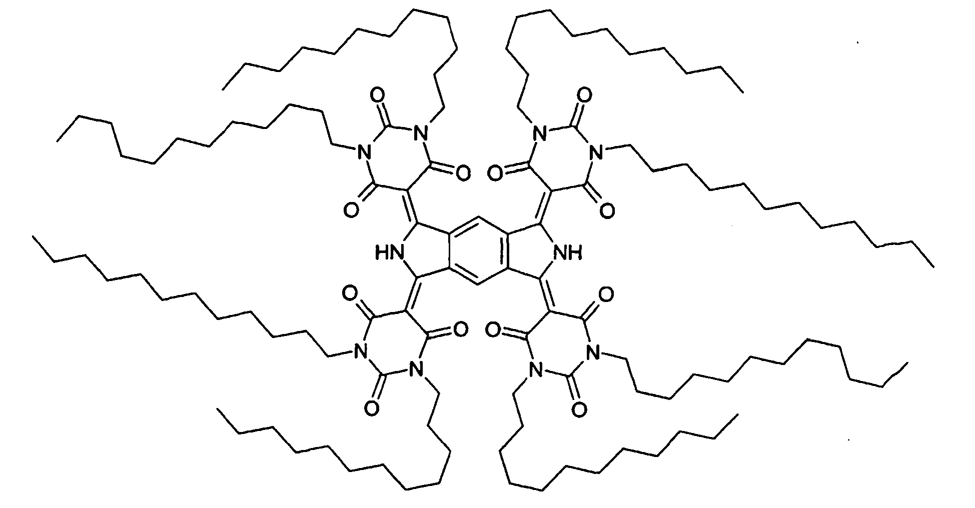 Schmelzpunkt: 207°C
MS (m/e): 2003 [M-H]⁻

### Beispiel 6: 2,2',2",2"'-Benzo[1,2-c:4,5-c']dipyrrol-1,3,5,7(2H,6H)-tetraylidentetrakis-1,3-dioxoindan

3,88 g Benzo[1,2-c:4,5-c']dipyrrol-1,3,5,7(2H,6H)-tetraimin und 16,1 g 1,3-Dioxoindan werden in 110 mL Eisessig erst 24 h bei Raumtemperatur gerührt und danach 6 h zum Sieden erhitzt. Die Suspension wird auf Raumtemperatur abgekühlt, filtriert, nacheinander mit Eisessig, Methanol und heißem Wasser (ca. 90°C) gewaschen und anschließend bei 60°C getrocknet. Die Ausbeute beträgt 12,9 g (97% d.Th.) eines braunroten Pulvers der folgenden Formel 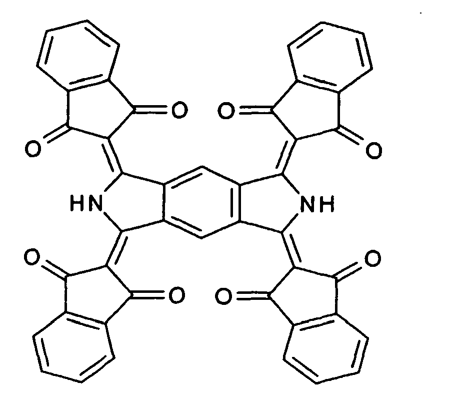 Schmelzpunkt: > 300°C
MS (m/e): 728 [M-H]⁻

### Beispiel 7: Umsetzung von Benzo[1,2-c:4,5-c']dipyrrol-1,3,5,7(2H,6H)-tetraimin mit Anilin und 2,4-Dihydroxychinolin

5,3 g Benzo[1,2-c:4,5-c']dipyrrol-1,3,5,7(2H,6H)-tetraimin und 8,1 g 2,4-Dihydroxychinolin werden in 150 ml Eisessig erst 24 Stunden bei Raumtemperatur gerührt. Nach Zugabe von 7,0 g Anilin wird 6 Stunden zum Sieden erhitzt. Die Suspension wird auf Raumtemperatur abgekühlt, filtriert, nacheinander mit Eisessig, Methanol und heißem Wasser (ca. 90°C) gewaschen und anschließend bei 60°C getrocknet. Die Ausbeute beträgt 11,0 g eines braunroten Pulvers eines Gemischs der folgenden Formeln 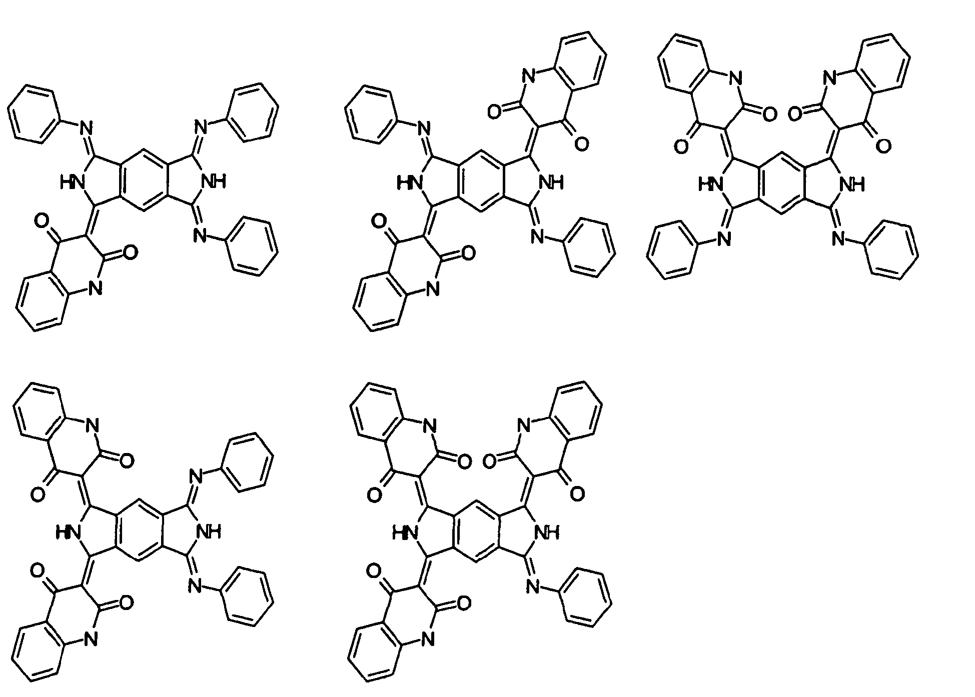 Schmelzpunkt: > 300°C
MALDI (m/e): 586, 654, 722 [M+H]⁺

### Beispiel 8: 5,5',5",5'"-Benzo[1,2-c:4,5-c']dipyrrol-1,3,5,7(2H,6H)-tetraylidentetrakis-1,3-diphenyl-2,4,6-trioxo-tetrahydropyrimidin

1,06 g Benzo[1.2-c:4,5-c']dipyrrol-1,3,5,7(2H,6H)-tetraimin und 8,41 g 1,3-Diphenylbarbitursäure werden in einer Mischung aus 10 mL Eisessig und 40 mL Toluol 1 h bei 50°C, dann 6 h am Rückfluss gerührt. Nach Abkühlen wird die Suspension filtriert, mit Eisessig, Methanol und heißem Wasser (ca. 90°C) gewaschen und bei 60°C getrocknet. Die Ausbeute beträgt 5,21 g (82% d.Th.) eines roten Pulvers der folgenden Formel 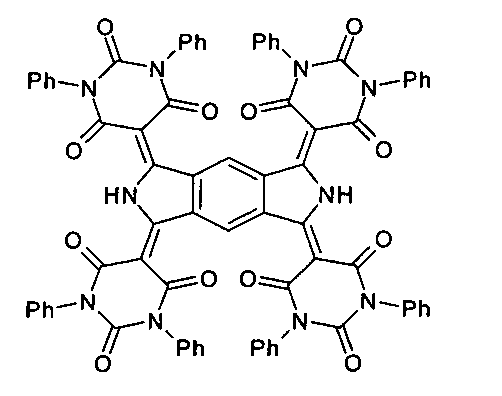 Schmelzpunkt: > 300°C
MS (m/e): 1266 [M+H]⁺, 1288 [M+Na]⁺

### Beispiel 9: 5,5',5",5"'-Benzo[1,2-c:4,5-c']dipyrrol-1,3,5,7(2H,6H)-tetraylidentetrakis-2,4,6-trioxo-tetrahydropyrimidin

Eine Suspension von 11,3 g 1,2,4,5-Tetracyanobenzol und 3,0 g Natriummethylatlösung (30 % in Methanol) in 340 mL Methanol wird 16 h bei RT gerührt. Nach Zugabe von 6,8 g Eisessig und 34,0 g Barbitursäure wird die Reaktionsmischung 24 h bei Raumtemperatur und anschließend 6 h zum Sieden erhitzt. Die Suspension wird auf Raumtemperatur abgekühlt und filtriert. Das Rohprodukt wird zur Reinigung 15 min in 250 mL Eisessig bei Siedetemperatur gerührt, heiß abgesaugt, nacheinander mit Eisessig und heißem Wasser (ca. 90°C) gewaschen und anschließend bei 60°C getrocknet. Die Ausbeute beträgt 38,2 g (92 % d.Th.) eines braunen Pulvers der folgenden Formel 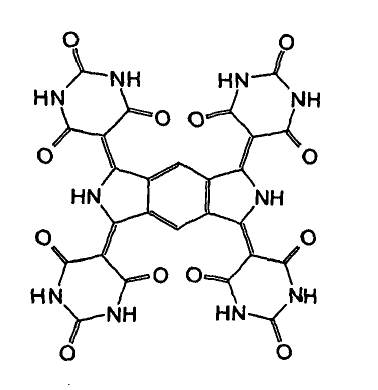 Schmelzpunkt: > 300°C
MALDI (m/e): 656 [M-H]⁻

### Anwendungsbeispiele

Zur Beurteilung der Eigenschaften der nach der Erfindung hergestellten Pigmente auf dem Lacksektor wurden aus der Vielzahl der bekannten Lacke ein aromatenhaltiger Alkydmelaminharzlack (AM) auf Basis eines mittelöligen Alkydharzes und eines butanolveretherten Melaminharzes, sowie ein aromatenfreier lufttrocknender Alkydharzlack (LA) auf Basis eines langöligen Sojaalkydharzes ausgewählt.

Zur Beurteilung der Eigenschaften der erfindungsgemäßen polymerlöslichen Farbstoffe wurde glasklares Polystyrol als einzufärbender Kunststoff ausgewählt. Die Herstellung von Prüfkörpern erfolgte durch Spritzgießen.

### Anwendungsbeispiel 1:

Eine Applikation des Pigments aus Beispiel 1a in AM-Lack zeigt im Vollton eine reine, blaustichig rote, in der Aufhellung eine farbstarke, rotstichig orangefarbene Lackierung.

### Anwendungsbeispiel 2:

Eine Applikation des Pigments aus Beispiel 1 b in AM-Lack zeigt im Vollton eine reine, blaustichig rote, in der Aufhellung eine farbstarke, rotstichig orangefarbene Lackierung.

### Anwendungsbeispiel 3:

Eine Applikation des Pigments aus Beispiel 2 in AM-Lack zeigt im Vollton und in der Aufhellung farbstarke, gelbstichig orangefarbene Lackierungen.

### Anwendungsbeispiel 4:

Eine Applikation des Pigments aus Beispiel 3 in LA-Lack ergibt im Vollton und in der Aufhellung gelbstichig orangefarbene Lackierungen.

### Anwendungsbeispiel 5:

Eine Applikation des Pigments aus Beispiel 3 in Polystyrol ergibt im Vollton und in der Aufhellung farbstarke rotstichig orangefarbene Prüfkörper.

### Anwendungsbeispiel 6:

Eine Applikation des Pigments aus Beispiel 4 in AM-Lack zeigt im Vollton eine reine rote, in der Aufhellung eine farbstarke, neutrale rote Lackierung.

### Anwendungsbeispiel 7:

Eine Applikation des Pigments aus Beispiel 4 in Polystyrol ergibt im Vollton und in der Aufhellung reine und farbstarke rotstichig orangefarbene Prüfkörper.

### Anwendungsbeispiel 8:

Eine Applikation des Pigments aus Beispiel 5 in Polystyrol ergibt im Vollton und in der Aufhellung reine und farbstarke rotstichig orangefarbene Prüfkörper.

### Anwendungsbeispiel 9:

Eine Applikation des Pigments aus Beispiel 6 in AM-Lack zeigt im Vollton eine deckende, gelbstichig rote, in der Aufhellung farbstarke, rotstichig braune Lackierung.

### Anwendungsbeispiel 10:

Eine Applikation des Pigments aus Beispiel 7 in AM-Lack zeigt im Vollton eine transparente braune, in der Aufhellung gelbstichig braune Lackierung.

### Anwendungsbeispiel 11:

Eine Applikation des Pigments aus Beispiel 8 in AM-Lack zeigt im Vollton ein deckendes Bordo, in der Aufhellung ein farbstarkes blaustichiges Rot.

### Anwendungsbeispiel 12:

Eine Applikation des Pigments aus Beispiel 9 in AM-Lack zeigt im Vollton eine deckend braune, in der Aufhellung neutrale braune Lackierung.

## Patentansprüche

1. Verbindung der allgemeinen Formel (I) 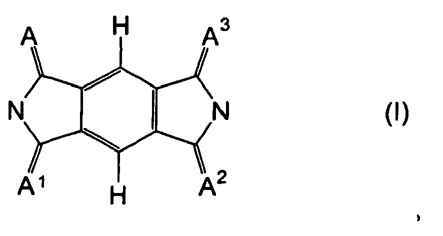 worin A für den zweiwertigen Rest einer cyclischen Verbindung der allgemeinen Formel (II) 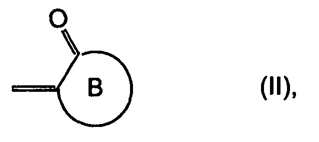 wobei B eine alicyclische oder heterocyclische Gruppe bedeutet, und A¹, A² und A³ gleich oder verschieden sind und die Bedeutung von A haben oder =NR bedeuten, wobei R Wasserstoff, unsubstituiertes oder durch 1, 2, 3 oder 4 Reste Halogen, R⁰, OR⁰, SR⁰, NH₂, NHR⁰, NR⁰₂, NO₂, COOH, COOR⁰, CONH₂, CONHR⁰, CONR⁰₂, CN, SO₃H, SO₂(OR⁰), SO₂R⁰, oder durch einen 5- bis 7-gliedrigen heteroaromatischen Rest mit 1, 2 oder 3 Heteroatomen aus der Gruppe N, O und S substituiertes Phenyl; einen 5- bis 7-gliedrigen heteroaromatischen Rest mit 1, 2 oder 3 Heteroatomen aus der Gruppe N, O und S; NH₂, NHR⁰, NR⁰₂, NHCONH₂ oder NHCONHR⁰ bedeutet, wobei R⁰ für C₁-C₁₈-Alkyl oder C₆-C₂₄-Aryl steht.

2. Verbindung nach Anspruch 1, **dadurch gekennzeichnet, dass** einer der Reste A¹, A² und A³ die Bedeutung von A hat.

3. Verbindung nach Anspruch 1, **dadurch gekennzeichnet, dass** zwei der Reste A¹, A² und A³ die Bedeutung von A haben.

4. Verbindung nach Anspruch 1, **dadurch gekennzeichnet, dass** A¹, A² und A³ die Bedeutung von A haben.

5. Verbindung nach mindestens einem der Ansprüche 1 bis 4, **dadurch gekennzeichnet, dass** A für den zweiwertigen Rest einer cyclischen Verbindung der allgemeinen Formel (III) steht 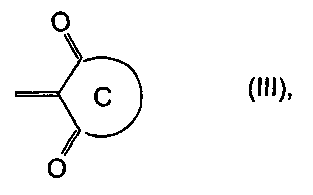 wobei C eine alicyclische oder heterocyclische Gruppe bedeutet.

6. Verbindung nach mindestens einem der Ansprüche 1 bis 5, **dadurch gekennzeichnet, dass** A einen zweiwertigen Rest der Formeln (a) bis (g) bedeutet, 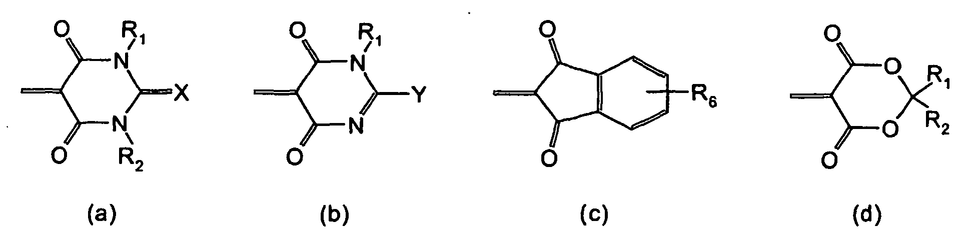 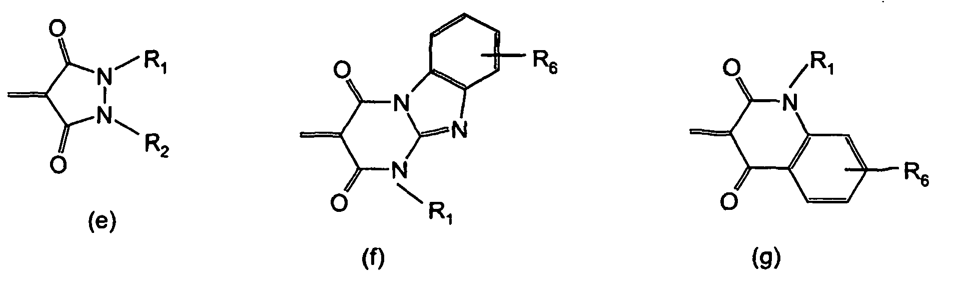 wobei R₁ und R₂ unabhängig voneinander für Wasserstoff, C₁-C₂₅-Alkyl, C₅-C₁₂-Cycloalkyl, C₆-C₂₄-Aryl, C₁-C₂₅-Alkyl-(C₆-C₁₀-aryl), einen 5- bis 7-gliedrigen heteroaromatischen Rest mit 1, 2 oder 3 Heteroatomen aus der Gruppe N, O und S, -(CH₂)ₙ-COR₃ oder -(CH₂)ₘ-OR₄, stehen,
worin R₃ für Hydroxy, Amino oder für unsubstituiertes oder ein- oder mehrfach mit Hydroxy oder Amino substituiertes C₁-C₂₅-Alkoxy, C₁-C₂₅-Alkylamino, Di-(C₁-C₂₅-alkyl)-amino, C₆-C₂₄-Arylamino, Di-(C₆-C₂₄-Aryl)-amino, C₁-C₂₅-Alkyl-(C₆-C₁₀-a ryl)-amino oder C₂-C₂₄-Alkenyloxy steht;
R₄ für Wasserstoff oder -CO-(C₁-C₂₅-Alkyl) steht, und
n und m unabhängig voneinander für eine ganze Zahl im Bereich von 0 bis 6 stehen,
und worin in R₁, R₂, R₃ und R₄ eine C-C- Einheit auch durch eine Ethereinheit C-O-C ersetzt sein kann;
X für =O, =S oder =NR₅ steht, worin R₅ die gleiche Bedeutung wie R₁ oder R₂ hat; Y für Wasserstoff, R₂, OR₂, SR₂, NHCN oder NR₂R₅ steht;
und R₆ Wasserstoff, Halogen, CN, R₂, OR₂, SR₂, NR₂R₅, NO₂, SO₂(OR₂), SO₂R₂, SO₂NR₂R₅ oder PO₂(OR₂) bedeutet.

7. Verbindung nach Anspruch 6, **dadurch gekennzeichnet, dass**
R₁ und R₂ Wasserstoff, C₁-C₁₈-Alkyl, C₅-C₆-Cycloalkyl, C₆-C₁₀-Aryl, Benzyl, Pyridyl, Pyrryl, Thienyl, Imidazolyl, Oxazolyl, Thiazolyl, Pyrimidyl, Hydroxycarbonyl-C₀-C₆-alkyl, C₁-C₁₈-Alkoxycarbonyl-C₀-C₆-alkyl, Aminocarbonyl-C₀-C₆-alkyl, C₁-C₁₈-Alkylaminocarbonyl-C₀-C₆-alkyl, C₆-C₁₀-Arylaminocarbonyl-C₀-C₆-alkyl, Di(C₁-C₁₈-alkyl)-aminocarbonyl-C₀-C₆-alkyl, C₁-C₁₈-Alkyl-C₆-C₁₀-arylaminocarbonyl-C₀-C₆-alkyl oder Di(C₆-C₁₀-aryl)-aminocarbonyl-C₀-C₆-alkyl bedeuten.

8. Verbindung nach Anspruch 6 oder 7, **dadurch gekennzeichnet, dass** R₃ Hydroxy, C₁-C₁₈-Alkoxy, C₁-C₁₈-Alkylamino, Di(C₁-C₁₈-alkyl)-amino, Benzylamino, C₆-C₁₀-Arylamino, Di(C₆-C₁₀-aryl)-amino oder (C₂-C₁₈)-Alkenyloxy bedeutet.

9. Verbindung nach einem oder mehreren der Ansprüche 6 bis 8, **dadurch gekennzeichnet, dass**
R₆ Wasserstoff, Cl, Br, C₁-C₁₈-Alkyl, C₅-C₆-Cycloalkyl, Benzyl, C₆-C₁₀-Aryl, Pyridyl, Pyrryl, Thienyl, Imidazolyl, Oxazolyl, Thiazolyl, Pyrimidyl, C₁-C₁₈-Alkoxy, C₆-C₁₀-Aryloxy, C₁-C₁₈-Alkylthio, C₆-C₁₀-Arylthio, C₁-C₁₈-Alkylamino, C₆-C₁₀-Arylamino, Di(C₁-C₁₈-alkyl)-amino, C₁-C₁₈-Alkyl-C₆-C₁₀-arylamino, Di(C₆-C₁₀-aryl)-amino, SO₃H, C₁-C₁₈-Alkoxysulfonyl, C₁-C₁₈-Alkylsulfonyl oder Di(C₁-C₁₈-alkyl)-aminosulfonyl bedeutet.

10. Verfahren zur Herstellung einer Verbindung nach mindestens einem der Ansprüche 1 bis 9, **dadurch gekennzeichnet, dass** 1,2,4,5-Tetracyanobenzol mit mindestens 2 Equivalenten Ammoniak und/oder Alkoholaten MOR₇,
worin M Natrium oder Kalium bedeutet und R₇ für C₁-C₁₈-Alkyl oder-(CH₂)ₘ-OH steht und m eine ganze Zahl im Bereich von 1 bis 6 bedeutet, und eine C-C-Einheit auch durch eine Ethereinheit C-O-C ersetzt sein kann,
in einem Lösemittel oder Lösemittelgemisch unter basischen bis neutralen Bedingungen bei einer Temperatur von -20 bis 120°C zu tetra-, tri-, di- oder monoiminosubstituierten Benzodipyrrolen umgesetzt wird,
die in einem Lösungsmittel oder Lösungsmittelgemisch unter neutralen bis sauren Bedingungen mit mindestens 1 Equivalent einer cyclischen Verbindung der Formel (VII), 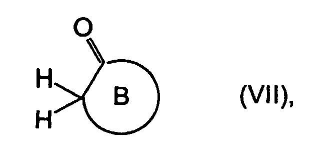 und gegebenenfalls höchstens 3 Equivalenten H₂NR umgesetzt werden.

11. Verwendung einer Verbindung nach mindestens einem der Ansprüche 1 bis 9 zum Einfärben oder Pigmentieren von organischen oder anorganischen, hoch-oder niedermolekularen Materialien.

12. Verwendung nach Anspruch 11 als Farbmittel für elektrophotographische Toner und Entwickler, für Farbfilter, für elektronische Tinten, sowie in optischen Schichten für die optische Datenspeicherung.

13. Zusammensetzung, enthaltend ein organisches oder anorganisches, hochmolekulares oder niedermolekulares Material, und mindestens eine der in einem oder mehreren der Ansprüche 1 bis 9 definierten Verbindungen in einer Menge von 0,005 bis 70 Gew.-%, bezogen auf das organische oder anorganische Material.

## Claims

1. A compound of the general formula (I) 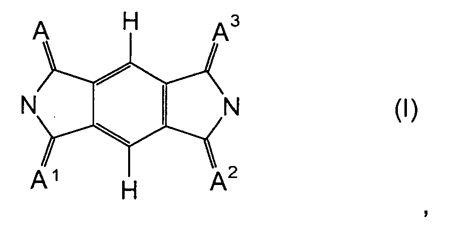 wherein A represents the divalent radical of a cyclic compound of the general formula (II) 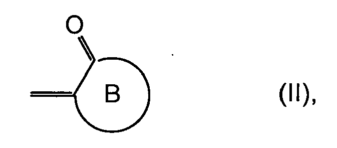 where B is an alicyclic or heterocyclic group, and
A¹, A² and A³ are the same or different and have the meaning of A or represent =NR, where R is hydrogen, unsubstituted phenyl, phenyl substituted by 1, 2, 3 or 4 radicals selected from the group consisting of halogen, R⁰, OR⁰, SR⁰, NH₂, NHR⁰, NR⁰₂, NO₂, COOH, COOR⁰, CONH₂, CONHR⁰, CONR⁰₂, CN, SO₃H, SO₂(OR⁰), SO₂R⁰, or by a 5- to 7-membered heteroaromatic radical having 1, 2 or 3 heteroatoms from the group consisting of N, O and S; a 5- to 7-membered heteroaromatic radical having 1, 2 or 3 heteroatoms from the group consisting of N, O and S; and NH₂, NHR⁰, NR⁰₂, NHCONH₂ or NHCONHR⁰, where R⁰ is C₁-C₁₈-alkyl or C₆-C₂₄-aryl.

2. The compound according to claim 1 wherein one of A¹, A² and A³ has the meaning of A.

3. The compound according to claim 1 wherein two of A¹, A² and A³ have the meaning of A.

4. The compound according to claim 1 wherein A¹, A² and A³ have the meaning of A.

5. The compound according to at least one of claims 1 to 4 wherein A represents the divalent radical of a cyclic compound of the general formula (III) 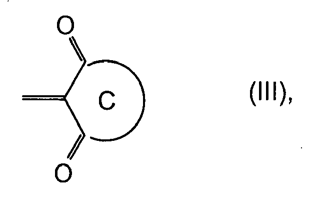 where C is an alicyclic or heterocyclic group.

6. The compound according to at least one of claims 1 to 5 wherein A is a divalent radical of the formulae (a) to (g) 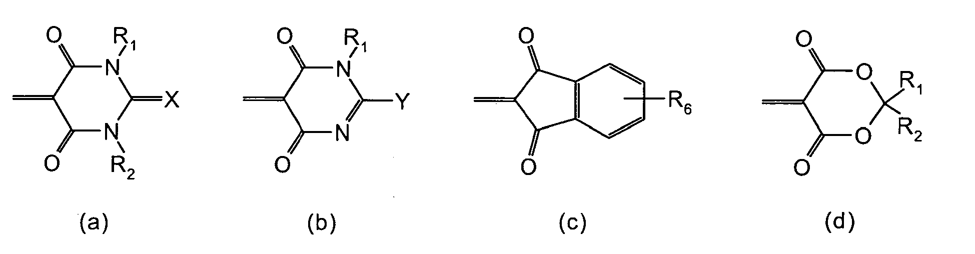 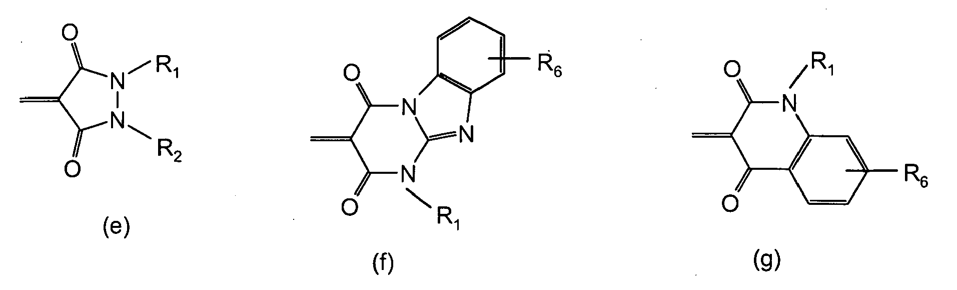 where R₁ and R₂ independently represent hydrogen, C₁-C₂₅-alkyl, C₅-C₁₂-cycloalkyl, C₆-C₂₄-aryl, C₁-C₂₅-alkyl-(C₆-C₁₀-aryl), a 5- to 7-membered heteroaromatic radical having 1, 2 or 3 heteroatoms from the group consisting of N, O and S, -(CH₂)ₙ-COR₃ or-(CH₂)ₘ-OR₄,
where R₃ is hydroxyl, amino or unsubstituted or singly or multiply hydroxyl- or amino-substituted C₁-C₂₅-alkoxy, C₁-C₂₅-alkylamino, di(C₁-C₂₅-alkyl)amino, C₆-C₂₄-arylamino, di(C₆-C₂₄-aryl)amino, C₁-C₂₅-alkyl-(C₆-C₁₀-aryl)amino or C₂-C₂₄-alkenyloxy;
R₄ is hydrogen or -CO-(C₁-C₂₅-alkyl), and
n and m are independently an integer from 0 to 6,
and where a C-C unit in R₁, R₂, R₃ and R₄ may also be replaced by an ether unit C-O-C;
X is =O, =S or =NR₅, where R₅ has the same meaning as R₁ or R₂;
Y is hydrogen, R₂, OR₂, SR₂, NHCN or NR₂R₅;
and R₆ is hydrogen, halogen, CN, R₂, OR₂, SR₂, NR₂R₅, NO₂, SO₂(OR₂), SO₂R₂, SO₂NR₂R₅ or PO₂(OR₂).

7. The compound according to claim 6 wherein
R₁ and R₂ are hydrogen, C₁-C₁₈-alkyl, C₅-C₆-cycloalkyl, C₆-C₁₀-aryl, benzyl, pyridyl, pyrryl, thienyl, imidazolyl, oxazolyl, thiazolyl, pyrimidyl, hydroxycarbonyl-C₀-C₆-alkyl, C₁-C₁₈-alkoxycarbonyl-C₀-C₆-alkyl, aminocarbonyl-C₀-C₆-alkyl, C₁-C₁₈-alkylaminocarbonyl-C₀-C₆-alkyl, C₆-C₁₀-arylaminocarbonyl-C₀-C₆-alkyl, di(C₁-C₁₈-alkyl)aminocarbonyl-C₀-C₆-alkyl, C₁-C₁₈-alkyl-C₆-C₁₀-arylaminocarbonyl-C₀-C₆-alkyl or di(C₆-C₁₀-aryl)aminocarbonyl-C₀-C₆-alkyl.

8. The compound according to claim 6 or 7 wherein
R₃ is hydroxyl, C₁-C₁₈-alkoxy, C₁-C₁₈-alkylamino, di(C₁-C₁₈-alkyl)amino, benzylamino, C₆-C₁₀-arylamino, di(C₆-C₁₀-aryl)amino or (C₂-C₁₈)-alkenyloxy.

9. The compound according to one or more of claims 6 to 8 wherein R₆ is hydrogen, Cl, Br, C₁-C₁₈-alkyl, C₅-C₆-cycloalkyl, benzyl, C₆-C₁₀-aryl, pyridyl, pyrryl, thienyl, imidazolyl, oxazolyl, thiazolyl, pyrimidyl, C₁-C₁₈-alkoxy, C₆-C₁₀-aryloxy, C₁-C₁₈-alkylthio, C₆-C₁₀-arylthio, C₁-C₁₈-alkylamino, C₆-C₁₀-arylamino, di(C₁-C₁₈-alkyl)amino, C₁-C₁₈-alkyl-C₆-C₁₀-arylamino, di(C₆-C₁₀-aryl)amino, SO₃H, C₁-C₁₈-alkoxysulfonyl, C₁-C₁₈-alkylsulfonyl or di(C₁-C₁₈-alkyl)aminosulfonyl.

10. A process for preparing a compound according to at least one of claims 1 to 9, which comprises reacting 1,2,4,5-tetracyanobenzene with at least 2 equivalents of ammonia and/or alkoxides MOR₇,
where M is sodium or potassium, R₇ is C₁-C₁₈-alkyl or-(CH₂)ₘ-OH and m is an integer from 1 to 6, and a C-C unit may also be replaced by an ether unit C-O-C, in a solvent or solvent mixture under basic to neutral conditions at a temperature in the range from -20 to 120°C to form tetra-, tri-, di- or monoimino-substituted benzodipyrroles,
which are subsequently, in a solvent or solvent mixture under neutral to acidic conditions, reacted with at least 1 equivalent of a cyclic compound of the formula (VII) 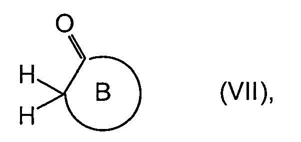 and if appropriate not more than 3 equivalents of H₂NR.

11. The use of a compound according to at least one of claims 1 to 9 for dyeing or pigmenting organic or inorganic, high or low molecular weight materials.

12. The use according to claim 11 as colorants for electrophotographic toners and developers, for color filters, for electronic inks and also in optical layers for optical data storage.

13. A composition comprising an organic or inorganic, high or low molecular weight material and at least one of the compounds defined in one or more of claims 1 to 9 in an amount from 0.005% to 70% by weight, based on the organic or inorganic material.

## Revendications

1. Composé de formule générale (I) 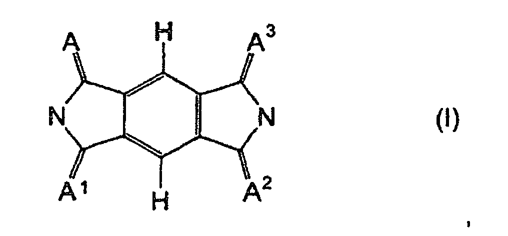 dans laquelle A représente le reste divalent d'un composé cyclique de formule générale (II) 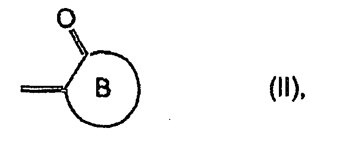 où B représente un groupe alicyclique ou hétérocyclique, et
A¹, A² et A³ sont identiques ou différents et ont la signification de A ou représentent =NR, R représentant un atome d'hydrogène, un radical phényle non substitué ou substitué par 1, 2, 3 ou 4 atomes d'halogène ou groupes R⁰, OR⁰, SR⁰, NH₂, NHR⁰, NR⁰₂, NO₂, COOH, COOR⁰, CONH₂, CONHR⁰, CONR⁰₂, CN, SO₃H, SO₂(OR⁰), SO₂R⁰, ou par un radical hétéroaromatique à 5-7 chaînons, comportant 1, 2 ou 3 hétéroatomes choisis parmi N, O et S ; un radical hétéroaromatique à 5-7 chaînons comportant 1, 2 ou 3 hétéroatomes choisis parmi N, O et S ; NH₂, NHR⁰, NR⁰₂, NHCONH₂ ou NHCONHR⁰, R⁰ représentant un groupe alkyle en C₁-C₁₈ ou aryle en C₆-C₂₄.

2. Composé selon la revendication 1, **caractérisé en ce que** l'un des radicaux A¹ A² et A³ a la signification de A.

3. Composé selon la revendication 1, **caractérisé en ce que** deux des radicaux A¹, A² et A³ ont la signification de A.

4. Composé selon la revendication 1, **caractérisé en ce que** A¹, A² et A³ ont la signification de A.

5. Composé selon au moins l'une des revendications 1 à 4, **caractérisé en ce que** A représente le reste divalent d'un composé cyclique de formule générale (III) 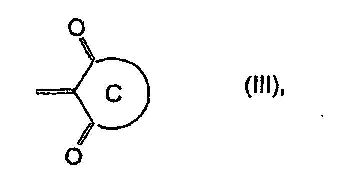 où C représente un groupe alicyclique ou hétérocyclique.

6. Composé selon au moins l'une des revendications 1 à 5, **caractérisé en ce que** A représente un radical divalent de formules (a) à (g), 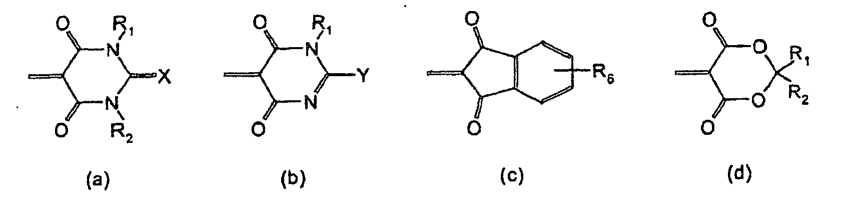 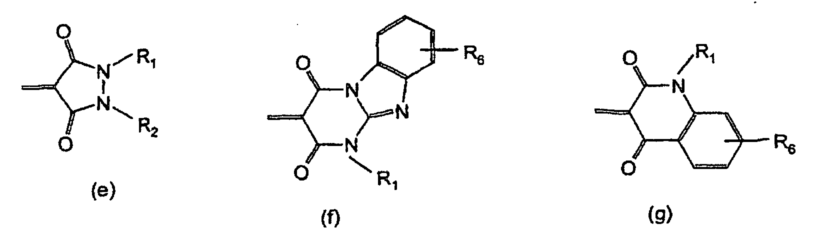 dans lesquelles R₁ et R₂ représentent, indépendamment l'un de l'autre, un atome d'hydrogène, un groupe alkyle en C₁-C₂₅, cycloalkyle en C₅-C₁₂, aryle en C₆-C₂₄, alkyl(C₁-C₂₅)-aryle(C₆-C₁₀), un radical hétéroaromatique à 5-7 chaînons, comportant 1, 2 ou 3 hétéroatomes choisis parmi N, O et S,-(CH₂)ₙ-COR₃ ou -(CH₂)ₘ-OR₄,
où R₃ représente un groupe hydroxy, amino ou un groupe alcoxy en C₁-C₂₅ non substitué ou une ou plusieurs fois substitué par hydroxy ou amino, un groupe alkyl(C₁-C₂₅)-amino, di [alkyl (C₁-C₂₅)]-amino, aryl (C₆-C₂₄) -amino, di[aryl(C₆-C₂₄)]-amino, alkyl(C₁-C₂₅)-[aryl(C₆-C₁₀)]-amino ou alcényloxy en C₂-C₂₄;
R₄ représente un atome d'hydrogène ou un groupe -CO-alkyle(C₁-C₂₅), et
n et m représentent, indépendamment l'un de l'autre, un nombre entier dans la plage allant de 0 à 6,
et où, dans R₁, R₂, R₃ et R₄ une unité C-C peut également être remplacée par une unité éther C-O-C;
X représente =O, =S ou =NR₅, R₅ ayant la même signification que R₁ ou R₂ ;
Y représente un atome d'hydrogène ou un groupe R₂, OR₂, SR₂, NHCN ou NR₂R₅ ;
et R₆ représente un atome d'hydrogène ou d'halogène ou un groupe CN, R₂, OR₂, SR₂, NR₂R₅, NO₂, SO₂(OR₂), SO₂R₂, SO₂NR₂R₅ ou PO₂(OR₂).

7. Composé selon la revendication 6, **caractérisé en ce que**
R₁- et R₂ représentent un atome d'hydrogène, un groupe alkyle en C₁-C₁₈, cycloalkyle en C₅-C₆, aryle en C₆-C₁₀, benzyle, pyridyle, pyrryle, thiényle, imidazolyle, oxazolyle, thiazolyle, pyrimidyle, hydroxycarbonylalkyle (C₀-C₆), alcoxy(C₁-C₁₈) arbonyl-alkyle(C₀-C₆), aminocarbonyl-alkyle(C₀-C₆), alkyl (C₁-C₁₈) aminocarbonyl-alkyle(C₀-C₆), aryl(C₆-C₁₀)aminocarbonylalkyle(C₀-C₆), di [alkyl (C₁-C₁₈)]-aminocarbonylalkyle(C₀-C₆), alkyl (C₁-C₁₈) -aryl (C₆-C₁₀) aminocarbonylalkyle(C₀-C₆) ou di[aryl(C₆-C₁₀)]-aminocarbonylalkyle(C₀-C₆).

8. Composé selon la revendication 6 ou 7,
**caractérisé en ce que**
R₃ représente un groupe hydroxy, alcoxy en C₁-C₁₈, alkyl (C₁-C₁₈) amino, di [alkyl(C₁-C₁₈)]-amino ; benzylamino, aryl (C₆-C₁₀) amino, di[aryl(C₆-C₁₀)]-amino ou alcényl(C₂-C₁₈)oxy.

9. Composé selon une ou plusieurs des revendications 6 à 8, **caractérisé en ce que**
R₆ représente un atome d'hydrogène, Cl, Br, un groupe alkyle en C₁-C₁₈, cycloalkyle en C₅-C₆, benzyle, aryle en C₆-C₁₀, pyridyle, pyrryle, thiényle, imidazolyle, oxazolyle, thiazolyle, pyrimidyle, alcoxy en C₁-C₁₈, aryloxy en C₆-C₁₀, alkyl(C₁-C₁₈)thio, aryl(C₆-C₁₀)thio, alkyl(C₁-C₁₈)amino, aryl(C₆-C₁₀)amino, di [alkyl (C₁-C₁₈)]-amino, alkyl(C₁-C₁₈)-aryl(C₆-C₁₀)-amino, di[aryl(C₆-C₁₀)]amino, SO₃H, alcoxy (C₁-C₁₈)sulfonyle, alkyl(C₁-C₁₈)sulfonyle ou di[alkyl (C₁-C₁₈)]-aminosulfonyle.

10. Procédé pour la préparation d'un composé selon au moins l'une des revendications 1 à 9, **caractérisé en ce qu'**on fait réagir du 1,2,4,5-tétracyanobenzène avec au moins 2 équivalents d'ammoniac et/ou d'alcoolates MOR₇, où M représente le sodium ou le potassium et R₇ représente un groupe alkyle en C₁-C₁₈ ou -(CH₂)ₘ-OH et m représente un nombre entier dans la plage de 1 à 6, et une unité C-C peut également être remplacée par une unité éther C-O-C, dans un solvant ou mélange de solvants, dans des conditions basiques à neutres, à une température de -20 à 120 °C, pour obtenir des benzodipyrroles tétra-, tri-, di- ou monosubstitués par imino,
que l'on fait réagir, dans un solvant ou mélange de solvants, dans des conditions neutres à acides, avec au moins 1 équivalent d'un composé cyclique de formule (VII), 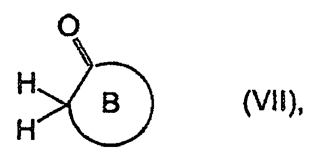 et éventuellement au maximum 3 équivalents de H₂NR.

11. Utilisation d'un composé selon au moins l'une des revendications 1 à 9, pour la coloration ou la pigmentation de matières organiques ou inorganiques, de faible masse moléculaire ou de masse moléculaire élevée.

12. Utilisation selon la revendication 11, en tant que colorant pour des toners et développeurs électrophotographiques, pour des filtres colorés, pour des encres électroniques, ainsi que dans des couches optiques pour la mémorisation optique de données.

13. Composition contenant une matière organique ou inorganique, de faible masse moléculaire ou de masse moléculaire élevée, et au moins l'un des composés définis dans une ou plusieurs des revendications 1 à 9, en une quantité de 0,005 à 70 % en poids, par rapport à la matière organique ou inorganique.
